# EUROPEAN PATENT APPLICATION

(11) **EP 1 801 092 A1**
(43) Date of publication of application: **27.06.2007**
(21) Application number: 06731939.2
(22) Date of filing: 11.04.2006
(51) Int. Cl.: C07C 51/353, C07C 63/331, C07B 61/00

(54) **PROCESS FOR PRODUCING BIPHENYLTETRACARBOXYLIC ACID**

(30) Priority: 14.04.2005 JP 2005116628
(71) Applicant: JFE Chemical Corporation, Tokyo 111-0051 (JP)
(72) Inventor: MORI, Hiroaki, c/o JFE CHEMICAL CORPORATION, Tokyo 111-0051 (JP)
(74) Representative: HOFFMANN EITLE
(86) International application number: PCT/JP2006/308003
(87) International publication number: WO 2006/112426

(57) **Abstract**

A method for producing a biphenyltetracarboxylic acid is to perform dimerization of a bromophthalate under the presence of a palladium catalyst and at least one reducing agent selected from the group consisting of saccharides and aliphatic monoalcohols having at least two carbon atoms. By this production method, since a side reaction is suppressed, a biphenyltetracarboxylic acid useful as a starting material for a polyimide resin having superior heat resistance and mechanical properties can be obtained in higher yield than ever.

## Description

### Technical Field

The present invention relates to a method for producing a biphenyltetracarboxylic acid.

### Background Art

A biphenyltetracarboxylic acid is useful as a main starting material for a polyimide resin having superior heat resistance and mechanical properties. As a method for producing a biphenyltetracarboxylic acid, heretofore, a dimerization reaction (dehalogeno-dimerization reaction) of a halogenophthalic acid has been known.

For example, in Japanese Unexamined Patent Application Publication No. 55-20705, a method has been disclosed in which dimerization is performed by placing a metalic palladium supported catalyst and methanol in an aqueous solution of an alkali metal salt of a 4-halogenophthalic acid. In addition, in Japanese Unexamined Patent Application Publication No. 62-26238, a dimerization method has been disclosed in which instead of methanol functioning as a reducing agent of the above reaction, a polyalcohol and/or a formaldehyde derivative is used. As is the above method, as a reducing agent of the above reaction, a method using formic acid or a formate has been disclosed in Japanese Unexamined Patent Application Publication No. 61-137838, and a method using carbon monoxide has been disclosed in Japanese Unexamined Patent Application Publication Nos. 61-293932 and 2-53742.

Furthermore, in Japanese Unexamined Patent Application Publication No. 6-122650, a method has been disclosed in which 4-chlorophthalate is used as a starting material, and the same dimerization as described above is performed under the presence of a saccharide. However, in this official gazette, the use of the saccharide is intended exclusively in consideration of the environment, and the effect as a reducing agent has not been described at all.

However, in the methods described above, in addition to the dimerization reaction of a halogenophthalic acid, a decomposition reaction of the halogenophthalic acid also competitively occurs. The dimerization reaction and the decomposition reaction are both carried out through a dehalogenation reaction, and the former produces a biphenyltetracarboxylic acid which is a target product; however, the latter produces phthalic acid as a by-product. That is, it can be said that the two reactions carried out through a dehalogenation reaction is rather regarded as a simultaneous reaction. Hence, by the conventional methods, since phthalic acid is produced as a by-product by the decomposition reaction, the yield of a desired biphenyltetracarboxylic acid cannot be disadvantageously increased.

An object of the present invention is to provide a method for producing a biphenyltetracarboxylic acid in higher yield than ever by controlling this simultaneous reaction.

### Disclosure of Invention

That is, the present invention relates to a method for producing a biphenyltetracarboxylic acid, including a step of performing dimerization of a bromophthalate under the presence of a palladium catalyst and at least one reducing agent selected from the group consisting of saccharides and aliphatic monoalcohols having at least two carbon atoms.

In the production method described above, the palladium catalyst is preferably a palladium-carbon catalyst. In this case, an amount of use of the palladium-carbon catalyst is preferably 10 to 100 parts by weight to 100 parts by weight of the bromophthalate, and the content of a palladium metal of the palladium-carbon catalyst is more preferably 1 to 10 parts by weight to 100 parts by weight of the catalyst.

In addition, in any of the production methods described above, the saccharides preferably include at least one selected from the group consisting of allose, altrose, glucose, mannose, gulose, galactose, talose, fructose, sorbose, and tagatose.

Furthermore, in any of the production methods described above, the aliphatic monoalcohols preferably include at least one selected from the group consisting of 1-propanol, 1-butanol, 2-methylpropanol, and 1-hexanol.

In addition, in any of the production methods described above, the dimerization reaction is preferably performed at a temperature in the range of 120 to 200°C.

Furthermore, the present invention relates to a method for producing a biphenyltetracarboxylic acid, including a step of performing dimerization of a bromophthalate under the presence of a palladium catalyst and, as a reducing agent, a saccharide and/or an aliphatic monoalcohol having at least two carbon atoms.

### Best Mode for Carrying Out the Invention

In order to achieve the above object, the inventor of the present invention carried out intensive research on a production method to obtain a biphenyltetracarboxylic acid in high yield. As a result, it was discovered that when a reaction is performed using a bromophthalate as a halogenophthalate under the presence of a palladium catalyst and, as a reducing agent, at least one selected from the group consisting of saccharides and aliphatic monoalcohols having at least two carbon atoms, the decomposition reaction of the halogenophthalate is suppressed, the dimerization reaction advantageously proceeds, and hence a biphenyltetracarboxylic acid can be obtained in higher yield than ever.

Next, with reference to preferable embodiments, the present invention will be described in more detail.

The method of the present invention uses a bromophthalic acid as a starting material. As the bromophthalic acid, 3-bromophthalic acid or 4-bromophthalic acid may be mentioned by way of example. As salts of the bromophthalic acids, although an alkali metal salt and/or an alkaline earth metal salt may be used, an alkali metal salt is preferably used. An alkali bromophthalate can be prepared by dissolving a bromophthalic acid in an aqueous alkali hydroxide solution containing lithium hydroxide, sodium hydroxide, potassium hydroxide, or the like.

As the reducing agent of the present invention, a saccharide and/or an aliphatic monoalcohol having at least two carbon atoms is used. In a conventional method for producing a biphenyltetracarboxylic acid, as a reducing agent, for example, formic acid, formaldehyde, methanol, polyalcohol, or carbon monoxide has been used. However, even when the reducing agent described above is used, the decomposition reaction of a bromophthalic acid cannot be suppressed, and as a result, many phthalic acid molecules are produced as a by-product. On the other hand, according to the present invention, a saccharide and/or an aliphatic monoalcohol having at least two carbon atoms is used as a reducing agent, and in addition, as a starting material, a bromophthalic acid is used in combination. Accordingly, the decomposition reaction of the bromophthalic acid can be suppressed.

As the saccharide used in the present invention, for example, there may be mentioned a monosaccharide, such as triose, tetorose, pentose, hexose, or heptose; a disaccharide, such as vicianose, primeverose, lactose, turanose, or rutinose; an oligosaccharide, such as fucosyllactose or sialyllactose; a polysaccharide; or a mixture thereof. Particularly preferable is a hexose which is a monosaccharide, and allose, altrose, glucose, mannose, gulose, galactose, talose, fructose, sorbose, or tagatose may be mentioned by way of example. An amount of use of the saccharide is 10 to 100 parts by weight to 100 parts by weight of a bromophthalate in a reaction solution and is preferably 20 to 80 parts by weight. When the saccharide in an amount of the lower limit or more is used, the decomposition reaction of a bromophthalic acid to phthalic acid can be easily suppressed. On the other hand, when the saccharide in an amount more than the upper limit is used, the productivity of biphenyltetracarboxylic acid is degraded, and hence it is not preferable from an economical point of view.

In the present invention, instead of the saccharide, as a reducing agent, an aliphatic alcohol having at least two carbon atoms may be used. In particular, an aliphatic monoalcohol having at least two carbon atoms is preferably used.

As the aliphatic monoalcohol having at least two carbon atoms used in the present invention, for example, ethanol, 1-propanol, 2-propanol, 1-butanol, 2-butanol, 2-methylpropanol, 1-hexanol, 2-hexanol, 3-hexanol, 2-ethylhexanol, or lauryl alcohol is preferably mentioned. Since the reaction is performed in an aqueous system, among those mentioned above, an aqueous alcohol having six carbon atoms or less is more preferable.

An amount of use of the aliphatic monoalcohol is 10 to 100 parts by weight to 100 parts by weight of a bromophthalate in a reaction solution and is preferably 20 to 80 parts by weight. When the alcohol in an amount of the lower limit or more is used, the decomposition reaction of a bromophthalic acid to phthalic acid can be easily suppressed. On the other hand, when the alcohol in an amount more than the upper limit is used, it is not preferable from an economical point of view. In addition, the saccharide and the aliphatic monoalcohol may be used alone or in combination.

As the palladium catalyst used in the method of the present invention, a palladium metal itself may be used, and in addition, a catalyst containing a palladium metal supported on a carrier, such as carbon, silica, alumina, silica-alumina, or titanium oxide, may also be mentioned by way of example. In particular, a palladium-carbon catalyst containing a palladium metal supported on carbon as a carrier is preferable. The above palladium catalysts have been known and are available from the market for the use in the present invention.

An amount of a supported palladium metal of the palladium-carbon catalyst is preferably 1 to 10 parts by weight to 100 parts of the catalyst and is more preferably 5 to 10 parts by weight. An amount of use of the palladium-carbon catalyst is preferably 10 to 100 parts by weight to 100 parts by weight of a bromophthalate and is more preferably 20 to 50 parts by weight. When the catalyst in an amount of the above lower limit or more is used, the conversion rate to biphenyltetracarboxylic acid by the dimerization reaction of a bromophthalic acid can be improved, and in addition, a side reaction in which a bromophthalic acid is decomposed to phthalic acid can be suppressed. On the other hand, when the catalyst in an amount more than the above upper limit is used, it is not preferable from an economical point of view.

In addition, although a reaction temperature of a conventional method for producing a biphenyltetracarboxylic acid is approximately 100°C or less, in the present invention, the reaction temperature is preferably set to 120 to 200°C. When the reaction temperature is set as described above, in addition to a decrease in amount of phthalic acid produced from a bromophthalic acid as a by-product, other side reactions such as polymerization can also be easily suppressed. A more preferable reaction temperature is in the range of 150 to 180°C.

A production method of the present invention is preferably performed in the range of atmospheric pressure to 10 MPa. In addition, in particular, a reaction of the present invention is preferably performed in the range of atmospheric pressure to 2 MPa in an inert gas atmosphere containing nitrogen, argon or the like.

Although the method for performing dimerization of a chlorophthalic acid under the presence of methanol functioning as a reducing agent has been disclosed (Japanese Unexamined Patent Application Publication No. 55-20705), when this reaction is performed at more than 100°C, a byproduction rate of phthalic acid caused by the decomposition of a halogenophthalic acid is increased. However, in a manner different from that of the methods described above, when an aliphatic monoalcohol having at least two carbon atoms is used as a reducing agent in the present invention, surprisingly, the production of phthalic acid as a by-product is rather suppressed at 100°C or more, in particular, at 120 to 200°C, and the yield of a biphenyltetracarboxylic acid is further improved. In the relationship between the temperature range and the boiling point of the aliphatic monoalcohol, the use of an aliphatic monoalcohol having a boiling temperature higher than that of methanol is effective.

A typical example of the method for producing a biphenyltetracarboxylic acid, according to the present invention, will be described below.

A predetermined amount of a solution prepared by dissolving a palladium-carbon catalyst and 4-bromophthalic anhydride in an aqueous sodium hydroxide solution and a predetermined amount of D-(+)-glucose or 1-butanol are sequentially added into an autoclave made of stainless steel. Next, after the system is purged with nitrogen and is then sealed in an air-tight manner, the system is heated to a predetermined temperature and is maintained for a predetermined time. After the reaction, the products are recovered, and the palladium-carbon catalyst is filtrated so as to be used again for another batch of the reaction.

Then, when the filtrate is processed by acid precipitation, a biphenyltetracarboxylic acid can be obtained. Furthermore, when the obtained precipitate is recrystallized, the biphenyltetracarboxylic acid can also be purified.

In the case in which the biphenyltetracarboxylic acid thus obtained is used as a starting material for a polyimide resin, anhydrization of it is further performed in accordance with a standard method, so that a biphenyltetracarboxylic dianhydride can be obtained.

### Examples

Next, the present invention will be described in detail with reference to examples and comparative examples. However, the present invention is not limited to those examples. In addition, "%" in the specification is on a mass basis unless particularly stated otherwise.

### [Example 1]

An aqueous solution of sodium 4-bromophthalate was prepared by sequentially dissolving 4.6 g (115.0 mmol) of sodium hydroxide, and 6.0 g (26.4 mmol) of 4-bromophthalic anhydride in 15 g of water. A palladium-carbon catalyst (a palladium metal supported rate of 5%, and a water content of 50%) in an amount of 4.4 g was added in an autoclave made of stainless steel having an inside volume of 50 ml, and subsequently, the above aqueous solution of sodium 4-bromophthalate and 2.4 g (13.3 mmol) of D-(+)-glucose were added. Next, after the system was purged with nitrogen, followed by air-tight sealing, the system was heated to 180°C and was then held for 5 hours at the same temperature. After the reaction, by performing a quantitative analysis using high-performance liquid chromatography, it was confirmed that sodium 3,4,3',4'-biphenyltetracarboxylate was produced. The reaction results are shown in Table 1.

### [Example 2]

Except that instead of D-(+)-glucose, D-(+)-galactose was used in an amount of the same moles as that of the glucose, the reaction was performed exactly in the same manner as that in Example 1. The reaction results are shown in Table 1.

### [Example 3]

Except that 4.3 g (58 mmol) of 1-butanol was used instead of D-(+)-glucose, the reaction was performed exactly in the same manner as that in Example 1. The reaction results are shown in Table 1.

### [Example 4]

Except that instead of 1-butanol, 2-methylpropanol was used in an amount of the same moles as that of the butanol, the reaction was performed exactly in the same manner as that in Example 3. The reaction results are shown in Table 1.

### [Example 5]

Except that 3.5 g (0.058 mol) of 1-propanol was used instead of 1-butanol, the reaction was performed exactly in the same manner as that in Example 3. The reaction results are shown in Table 1.

### [Example 6]

Except that 6.2 g (60 mmol) of 1-hexanol was used instead of 1-butanol, the reaction was performed exactly in the same manner as that in Example 3. The reaction results are shown in Table 1.

### [Comparative Example 1]

Except that 4.8 g (26.3 mmol) of 4-chlorophthalic anhydride was used instead of 4-bromophthalic anhydride, the reaction was performed exactly in the same manner as that in Example 1. The reaction results are shown in Table 1.

### [Comparative Example 2]

Except that 2.4 g (38.7 mmol) of ethylene glycol was used instead of D-(+)-glucose, the reaction was performed exactly in the same manner as that in Example 1. The reaction results are shown in Table 1.

### [Comparative Example 3]

Except that 4.8 g (26.3 mmol) of 4-chlorophthalic anhydride was used instead of 4-bromophthalic anhydride, and that 2.4 g (38.7 mmol) of ethylene glycol was used instead of D-(+)-glucose, the reaction was performed exactly in the same manner as that in Example 1. The reaction results are shown in Table 1.

### [Comparative Example 4]

Except that 4.8 g (26 mmol) of 4-chlorophthalic anhydride was used instead of 4-bromophthalic anhydride, and that 3.6 g (112 mmol) of methanol was used instead of 1-butanol, the reaction was performed exactly in the same manner as that in Example 3. The reaction results are shown in Table 1.

According to many conventional methods for producing a biphenyltetracarboxylic acid, although a halogenophthalic acid is used as a starting material, a chlorophthalic acid is actually used. From the examples and the comparative examples in Table 1, it is understood that when a bromophthalic acid is used instead of a chlorophthalic acid, and a saccharide and/or an aliphatic monoalcohol having at least two carbon atoms is used as a reducing agent, a biphenyltetracarboxylic acid can be obtained in high yield. That is, it is understood that by the method of the present invention, a competitive decomposition reaction of a bromophthalic acid is suppressed, and that the dimerization reaction of the bromophthalic acid is dominantly performed.

**Table 1**

| | Halogenophthalic acid | Reducing Agent | Conversion Rate mol% | Selection Rate mol% | Yield mol% |
|---|---|---|---|---|---|
| Example 1 | 4-BPA | D-(+)-Glucose | 100 | 88.0 | 88.0 |
| Example 2 | 4-BPA | D-(+)-Galactose | 100 | 87.1 | 87.1 |
| Example 3 | 4-BPA | 1-Butanol | 100 | 79.6 | 79.6 |
| Example 4 | 4-BPA | 2-Methylpropanol | 100 | 77.4 | 77.4 |
| Example 5 | 4-BPA | 1-Propanol | 100 | 77.7 | 77.7 |
| Example 6 | 4-BPA | 1-Hexanol | 98.7 | 75.3 | 74.3 |
| Comparative Example 1 | 4-CPA | D-(+)-Glucose | 100 | 53.1 | 53.1 |
| Comparative Example 2 | 4-BPA | Ethylene Glycol | 98.4 | 61.8 | 60.8 |
| Comparative Example 3 | 4-CPA | Ethylene Glycol | 98.1 | 43.2 | 42.4 |
| Comparative Example 4 | 4-CPA | Methanol | 100 | 41.6 | 41.6 |

| | | | | | |
|---|---|---|---|---|---|
| • 4-BPA: Sodium 4-bromophthalate • 4-CPA: Sodium 4-chlorophthalate • Conversion Rate/mol%: (consumed halogenophthalate/used halogenophthalate)×100 • Selection Rate/mol%: 2×(biphenyltetracarboxylate/consumed halogenophthalate)×100 • Yield/mol%: 2×(biphenyltetracarboxylate/used halogenophthalate)×100 | | | | | |

### Industrial Applicability

By the method of the present invention in which a dimerization reaction of a bromophthalate is performed under the presence of a palladium catalyst with a reducing agent selected from a saccharide and/or an aliphatic monoalcohol having at least two carbon atoms, a side reaction such as decomposition of a starting material can be suppressed, and hence a biphenyltetracarboxylic acid can be obtained in higher yield than ever. A biphenyltetracarboxylic acid is useful as a main starting material for a polyimide resin having superior heat resistance and mechanical properties, and the present invention widely contributes to industries.

## Claims

1. A method for producing a biphenyltetracarboxylic acid, comprising a step of:
performing dimerization of a bromophthalate under the presence of a palladium catalyst and at least one reducing agent selected from the group consisting of saccharides and aliphatic monoalcohols having at least two carbon atoms.

2. The method for producing a biphenyltetracarboxylic acid, according to Claim 1, wherein the palladium catalyst is a palladium-carbon catalyst.

3. The method for producing a biphenyltetracarboxylic acid, according to Claim 2, wherein the amount of use of the palladium-carbon catalyst is 10 to 100 parts by weight to 100 parts by weight of the bromophthalate, and the content of a palladium metal of the palladium-carbon catalyst is 1 to 10 parts by weight to 100 parts by weight of the catalyst.

4. The method for producing a biphenyltetracarboxylic acid, according to Claim 1, wherein the saccharide is at least one selected from the group consisting of allose, altrose, glucose, mannose, gulose, galactose, talose, fructose, sorbose, and tagatose.

5. The method for producing a biphenyltetracarboxylic acid, according to Claim 1, wherein the aliphatic monoalcohol is at least one selected from the group consisting of 1-propanol, 1-butanol, 2-methylpropanol, and 1-hexanol.

6. The method for producing a biphenyltetracarboxylic acid, according to one of Claims 1 to 5, wherein the dimerization reaction is performed at a temperature in the range of 120 to 200°C.

7. A method for producing a biphenyltetracarboxylic acid, comprising a step of performing dimerization of a bromophthalate under the presence of a palladium catalyst and, as a reducing agent, a saccharide and/or an aliphatic monoalcohol having at least two carbon atoms.
